# EUROPÄISCHE PATENTSCHRIFT

(11) **EP 0 529 035 B1**
(45) Veröffentlichungstag und Bekanntmachung des Hinweises auf die Patenterteilung: **28.05.1997**
(21) Anmeldenummer: 92905902.0
(22) Anmeldetag: 12.03.1992
(51) Int. Cl.: A61L 2/06, A61L 2/22, A61H 33/00

(54) **VERFAHREN ZUR DESINFEKTION DES ROHRLEITUNGSSYSTEMS AN EINEM WHIRLPOOL UND WHIRLPOOL ZUR DURCHFÜHRUNG DES VERFAHRENS**
PROCESS FOR DISINFECTING PIPEWORK SYSTEMS IN A WHIRLPOOL AND WHIRLPOOL FOR IMPLEMENTING SAID PROCESS
PROCEDE POUR LA DESINFECTION DE SYSTEMES DE TUYAUTERIE DANS UNE INSTALLATION DE BAIN A REMOUS ET BAIN A REMOUS POUR LA REALISATION DU PROCEDE

(30) Priorität: 15.03.1991 DE 4108539
(43) Veröffentlichungstag der Anmeldung: 03.03.1993
(73) Patentinhaber: HOESCH Metall + Kunststoffwerk GmbH & Co., 52304 Düren (DE)
(72) Erfinder: ESSER, Hans-Peter, D-5010 Bergheim (DE)
(74) Vertreter: Langmaack, Jürgen, Dipl.-Ing.
(86) Internationale Anmeldenummer: EP9200549
(87) Internationale Veröffentlichungsnummer: WO9216243

(56) Entgegenhaltungen:
- EP-A- 0 297 246
- DE-A- 3 928 464
- FR-A- 1 122 539
- FR-A- 2 603 976
- US-A- 1 491 089
- US-A- 4 944 919

## Beschreibung

Die Erfindung betrifft ein Verfahren zur Desinfektion des Rohrleitungssystems an einem Whirlpool entsprechend dem Oberbegriff des Anspruchs 1.

Die Desinfektion der Rohrsysteme von Whirlpool-Wannen wurde je nach Bauart bisher entweder beim Befüllen der Wanne vorgenommen, wobei während des Füllvorganges ein Teil des Frischwassers aus der Wanne rückwärts ablaufend über das Rohrsystem unmittelbar in das Abflußrohr abgeführt wurde. Durch die Anordnung von Sperrventilen an den Wirbeldüsen ergab sich darüber hinaus die Möglichkeit, das Rohrleitungssystem mit Hilfe der Umwälzpumpe über eine kurze Zeit mit Druck durchzuspülen, wobei druckseitig das Wasser dann nicht in die Wanne sondern in das Abflußrohr abgegeben wurde (DE-OS 34 20 714).

Bei anderen Verfahren wird nach Abschluß des Badevorganges unmittelbar nach Ablauf der Wannenfüllung mit einem Desinfektionsmittel versetztes Frischwasser durch das Rohrsystem praktisch drucklos hindurchgeführt, wobei hier jedoch zum einen der Nachteil besteht, daß relativ große Frischwassermengen benötigt werden und zum anderen das mit Desinfektionsmitteln versetzte Wasser auch in den Wanneninnenraum gelangt. Bei diesem Verfahren muß in einem zweiten Schritt das gesamte Rohrsystem einschließlich des Wanneninnenraums sorgfältig mit reinem Frischwasser durchspült werden, um Hautbeeinträchtigungen beim nächsten Bad zu vermeiden (DE-OS 37 22 281).

Schließlich wurde versucht, fest verschließbare Düsen zu konzipieren, um unter Umkehr der Durchströmungsrichtung das Rohrsystem mit Hilfe der Umwälzpumpe mit dem vollen Pumpendruck durchspülen zu können, so daß hier unter Ausnutzung der mechanischen Wirkung der Wasserströmung die Bildung von Ablagerungen im Rohrsystem vermieden wurde. Das Problem besteht hierbei insbesondere darin, daß im Bereich der Verschlußmechanismen für die Düsen keine Einwirkung der Wasserströmung möglich ist (DE-PS 39 02 117).

Aus FR 2 603 976 A1 ist es bekannt, in einem Druckbehälter überhitzten Dampf unter Druck zur Verwendung im Haushalt zu erzeugen und an der Verwendungsstelle unter hohem Druck auszublasen. Durch Einsprühen einer Flüssigkeit wird dann die Temperatur reduziert und die zu erzeugende Heißdampfmenge reduziert.

Aus US-A-1 491 089 ist eine Badewanne bekannt, in die eine Spannliege mit Abstand über dem Wannenboden eingehängt ist und bei der eine auf der Spannliege befindliche Person mit einer Haube abgedeckt ist, die nur den Kopf freiläßt. In den Raum unterhalb der Spannliege wird Warmluft und Dampf eingeführt, die sich dort vermischen und dann als Warmluft-Dampf-Gemisch durch die Öffnungen der Spannliege strömen und hierbei den Körper der Person umspülen. Die Temperatur des Luft-Dampf-Gemisches ist so eingestellt, daß sie in etwa den üblichen Temperaturen eines Dampfbades entsprechen, d. h. unterhalb von 50° C liegen.

Aus FR-A-1 122 539 ist ein Dampferzeuger bekannt, bei dem auf einen mit Heizelementen aufgeheizten Verdampferkörper Wasser aufgebracht wird, das verdampft und in Dampfform abgezogen wird.

Aus DE-A-3 928 464 ist eine Whirlpool-Wanne bekannt, die nach dem Reinigen des Rohrsystems mit Wasser mit Heißluft nachgespült wird. Mit diesem Nachspülen soll das in dem Rohrleitungssystem befindliche Restwasser getrocknet werden, so daß eine Vermehrung im Rohrleitungssystem verbliebener Keime unterbunden wird. Eine Desinfektion ist hierdurch nicht möglich.

Der Erfindung liegt nun die Aufgabe zugrunde, ein Verfahren zur Desinfektion des Rohrleitungssystems an einem Whirlpool zu schaffen, mit dem die vorstehend geschilderten Nachteile vermieden werden.

Diese Aufgabe wird erfindungsgemäß mit den im Kennzeichungsteil des Anspruchs 1 angegebenen Verfahrensschritten gelöst.

Der Begriff "nebelförmiges Medium" im Sinne der vorliegenden Erfindung umfaßt einen mit Hilfe einer Flüssigkeit unter Temperatureinwirkung erzeugten, in einen Trägerstrom eingebrachten Naßdampf oder Sattdampf, sowie auch einen Nebel oder ein Aerosol, d. h. eine in ein aufgeheiztes Trägergas eingebrachte Flüssigkeit in feinster Verteilung. Überraschend hat sich nämlich gezeigt, daß beispielsweise eine mit Pseudomonas aeruginosa kontaminierte Rohrleitung nach einer Beaufschlagung mit Wasserdampf als nebelförmigem Medium mit einer Temperatur zwischen 70° und 90°C über einen Zeitraum von nur wenigen Minuten bakterienfrei war.

Der besondere Vorteil dieses Verfahrens besteht darin, daß anstelle von großen Spülwassermengen und der Verwendung von Chemikalien hier lediglich eine geringe Flüssigkeitsmenge und geringe Energiemengen zur Erzeugung des nebelförmigen Mediums erforderlich ist. Das nebelförmige Medium kann hierbei in einfacher Weise durch das gesamte Rohrsystem geführt werden und hierbei auch sowohl die Wirbeldüsen frei bis in den Wanneninnenraum durchströmen, als auch über die Druckleitung, die Umwälzpumpe und die Ansaugöffnung bis in den Wanneninnenraum durchströmen. Damit werden alle für eine Kontamination kritischen Bereiche einer Whirlpool-Wanne der Einwirkung des aufgeheizten nebelförmigen Mediums ausgesetzt.

Im Rahmen des erfindungsgemäßen Verfahrens wird das nebelförmige Medium durch Verdampfen einer Flüssigkeit unter Temperatureinwirkung erzeugt. Hierzu wird in erste Linie Wasser, vorzugsweise entmineralisiertes Wasser, verwendet. Bevorzugt wird hierbei ein Verdampfungsvorgang unter normalem Atmosphärendruck. Dies hat den Vorteil, daß für die Erzeugung des nebelförmigen Mediums ein elektrisch beheizter drucklos arbeitender Dampfgenerator eingesetzt werden kann, da für die Lösung der gestellten Aufgabe geringe Dampfleistungen benötigt werden. Der Vorteil liegt hierbei darin, daß derartige Dampfgeneratoren keine Druckbehälter aufweisen und dementsprechend nicht als Dampfkessel im technischen Sinne gelten. Die Durchströmung des Rohrsystems erfolgt hierbei über die vom Dampfgenerator erzeugte Volumenverdrängung. Ein besonderer Vorteil der Verwendung von heißem Wasserdampf besteht darin, daß eine Entkeimung des Wassers durch den Verdampfungsvorgang erfolgt, so daß hier von außen keine neuen Keime in das Rohrsystem eingetragen werden, auch dann nicht, wenn der erzeugte Wasserdampf mit einer Temperatur von weniger als 100°C in das Rohrsystem eingeleitet wird. Ein weiterer Vorteil dieser Verfahrensweise besteht bei der Behandlung eines Whirlpools darin, daß anstelle von 30 bis 40 l Wasser, wie es bisher zum Spülen der Rohrsysteme ggf. mit Zusatz von Desinfektionsmitteln benötigt wurde, nur eine Wassermenge je nach Größe der Anlage zwischen 1 und 3 l benötigt wird.

Zur Erzeugung des nebelförmigen Mediums wird eine Flüssigkeit in feinster Verteilung zur Bildung eines Aerosols in ein Trägergas eingebracht. Diese Verfahrensweise bietet die Möglichkeit, daß der für die Durchströmung des Rohrsystems notwendige Volumenstrom über das Trägergas bereitgestellt wird, wobei die Durchströmungsgeschwindigkeit ggf. über einen entsprechenden Druckerzeuger, beispielsweise ein Gebläse, erzeugt wird und daß die Flüssigkeit genau dosiert in den Trägergasstrom in feinster Verteilung eingesprüht wird. Die für die Desinfektion erforderliche notwendige Temperaturlage des durch das Einsprühen von Flüssigkeit in das Trägergas gebildeten nebelförmigen Mediums kann nun in einer Ausgestaltung der Erfindung dadurch bewirkt werden, daß das Trägergas vor Bildung des Aerosols aufgeheizt wird. Hier muß jedoch je nach der pro Zeiteinheit in den Trägergasstrom eingedüsten Flüssigkeitsmenge mit einer höheren Temperatur für den Trägergasstrom gearbeitet werden, weil die Eindüsung der Flüssigkeit zu einer Temperaturabsenkung nach Art einer Verdampfungskühlung wirkt. Die Temperaturlage kann jedoch genau eingestellt werden, so daß das erzeugte nebelförmige Medium mit einer genau vorgebbaren Temperatur in das Rohrsystem eingeleitet werden kann.

In einer Ausgestaltung der Erfindung ist vorgesehen, daß das gebildete Aerosol vor der Einleitung in das Rohrsystem aufgeheizt wird. Dieser Verfahrensschritt kann je nach Ausgestaltung der Temperaturregelung und der Mengenregelung für die einzudüsende Flüssigkeit auch mit dem voraufgegangenen Verfahrensschritt, d.h. einer Aufheizung des Trägergasstroms vor Bildung des Aerosols kombiniert werden. Besonders zweckmäßig ist es jedoch, wenn die Flüssigkeit auf eine vorzugsweise temperaturregelbare Verdampferfläche aufgesprüht wird. Dies hat den Vorteil, daß zum einen der Trägergasstrom aufgeheizt wird und daß die Verdampfungsleistung nicht vom Trägergasstrom sondern von der Verdampferfläche aufgebracht wird. Die vorherige Versprühung oder Zerstäubung der Flüssigkeit beschleunigt die Verdampfung und erlaubt eine bessere Regelung.

Bei der Verwendung von Wasser zur Erzeugung des nebelförmigen Mediums durch Bildung eines Aerosols muß jedoch darauf geachtet werden, daß das Wasser selbst keimfrei ist, da bei dieser Verfahrensweise in der Regel mit Temperaturen gearbeitet wird, die unterhalb der Siedetemperatur des Wassers liegen, so daß die Entkeimung der verwendeten Flüssigkeit selbst während des Vorgangs der Aerosolbildung nicht mit der gleichen Sicherheit gegeben ist, wie dies bei der Bildung des nebelförmigen Mediums durch Dampf gegeben ist.

In weiterer Ausgestaltung des erfindungsgemäßen Verfahrens ist vorgesehen, daß als Flüssigkeit zur Erzeugung des nebelförmigen Mediums, zumindest als Zusatz zu Wasser, ein temperaturbeständiges Desinfektionsmittel verwendet wird. Diese Ausgestaltung des Verfahrens hat den Vorteil, daß zusätzlich zu der thermischen Einwirkung auch noch eine chemische Einwirkung erfolgt. Dies ist insbesondere dann zweckmäßig, wenn die maximal zulässige Temperatur durch das Material der zu desinfizierenden Oberflächen beschränkt ist, beispielsweise eine Temperatur von 80°C nicht überschritten werden darf, gleichwohl damit zu rechnen ist, daß Keime vorhanden sind, die bei dieser Temperaturlage mit Sicherheit noch nicht abgetötet sind. Voraussetzung ist jedoch, daß das Desinfektionsmittel sich bei einer Erhitzung auf derartige Temperaturen chemisch nicht zersetzt. Weitere Voraussetzung ist, daß das verwendete Desinfektionsmittel in seiner Dampfform, auch vermischt mit Wasserdampf, beim Einatmen nicht gesundheitsschädlich ist.

Die Erfindung betrifft ferner einen Whirlpool zur Durchführung des erfindungsgemäßen Verfahrens mit einem Becken, dessen Wandungen und/oder Boden mit Düsen zur Einleitung von Wasser und/oder Luft versehen sind, die über Rohrleitungen mit wenigstens einem Druckerzeuger zur Beaufschlagung der Düsen mit Wasser und/oder Luft verbunden sind. Eine derartige meist in Form einer Wanne ausgebildete Whirlpool-Anlage arbeitet mit einer Umwälzpumpe, die Wasser aus dem Wanneninnenraum über eine Saugleitung ansaugt und dann über eine Druckleitung, mit der mehrere in den Wanneninnenraum gerichtete Düsen verbunden sind, das Wasser wieder in den gefüllten Wanneninnenraum eindrückt. Hierbei kann zusätzlich von dem Wasserstrahl über die Düse Luft angesaugt werden, so daß in den Wanneninnenraum ein Strahl aus einem Wasser-Luft-Gemisch eintritt. Anstelle derartiger Wirbeldüsen oder auch zusätzlich zu derartigen Wirbeldüsen können vorzugsweise im Bodenbereich Düsen angeordnet sein, die über eine Druckleitung mit einem Gebläse verbunden sind, so daß in die Wannenfüllung von unten über eine Vielzahl von Düsen Luft eingeleitet wird. Dieses an sich nur mit Luft beaufschlagte Rohrleitungssystem kommt mit der Wannenfüllung dann in Berührung, wenn der Druckerzeuger abgeschaltet und/oder nach Beendigung des Badebetriebes die Wannenfüllung abgelassen wird, wobei ein Teil der Wannenfüllung über die Luftleitungen, die über ein Ventil gegenüber dem Abflußrohr abgesperrt sind, dann in das Abflußrohr entleert werden.

Erfindungsgemäß ist für einen derartigen Whirlpool vorgesehen, daß an eine zentrale Druck- und/oder Saugleitung im Bereich des Druckerzeugers eine Zuleitung einmündet, die mit einer Einrichtung zur Erzeugung eines aufgeheizten nebelförmigen strömungsfähigen Mediums verbunden ist. Mit Hilfe einer derartigen Anordnung ist es möglich, durch das gesamte Rohrleitungssystem einschließlich der Umwälzpumpe das aufgeheizte nebelförmige Medium hindurchzuführen, wobei das Medium aus den Düsenöffnungen ebenso wie aus den Ansaugöffnungen der Umwälzpumpe sowie aus den im Boden befindlichen reinen Luftdüsen austreten kann. Damit ist gewährleistet, daß alle Teile, die mit dem gebrauchten Badewasser in Berührung gekommen sind und die von Hand nicht zugänglich sind, der desinfizierenden Wirkung des aufgeheizten nebelförmigen Mediums ausgesetzt werden. Es muß lediglich dafür Sorge getragen werden, daß über eine Sperrvorrichtung die Einmündung der Druckleitung des Druckerzeugers für Luft für den Desinfektionsvorgang abgesperrt wird, da dem Druckerzeuger für die Luft in der Regel eine elektrische Heizpatrone unmittelbar nachgeschaltet ist, die mit dem nebelförmigen Medium nicht in Berührung kommen darf. Diese ist jedoch andererseits auch so angeordnet, daß die Druckleitung in diesem Bereich mit dem verbrauchten Badewasser zu keinem Zeitpunkt in Berührung kommt.

Hierbei ist eine Einrichtung zur Erzeugung eines nebelförmigen Mediums vorgesehen, die ein an die Zuleitung angeschlossenes Gehäuse aufweist, das mit wenigstens einer Heizeinrichtung zur Aufheizung von Gebläseluft und mit einem Gebläse in Verbindung steht und wenigstens eine Sprühvorrichtung zur Erzeugung feinster Tröpfchen, die vorzugsweise in das Gehäuse im Bereich der Heizeinrichtung mündet. Die Einmündung der Sprühvorrichtung in die Zuleitung kann auch, in Strömungsrichtung der Luft gesehen, entweder vor der Heizeinrichtung oder hinter der Heizeinrichtung erfolgen. Ggf. kann zur Vereinfachung der Regelung eine zweite Heizeinrichtung vorgesehen werden, so daß die Flüssigkeit in aufgeheizte Luft eingesprüht wird und dann das aufgeheizte nebelförmige Medium über eine zweite Heizeinrichtung auf die endgültige Behandlungstemperatur hochgeheizt wird.

Bevorzugt ist in einer Ausgestaltung vorgesehen, daß der Sprühstrahl gegen eine Verdampferfläche der Heizeinrichtung gerichtet ist. Hierdurch wird erreicht, daß der Trägergasstrom, d. h. die über ein Gebläse zugeführte Luft aufgeheizt werden kann und daß die für die Verdampfung notwendige Heizenergie nicht wieder dem Trägergas entnommen sondern direkt über die Verdampferfläche in die aufgesprühte Flüssigkeit eingeleitet wird, so daß es zu einer sehr schnellen Verdampfung nach Art einer Filmverdampfung kommt.

Da nur eine geringe Trägergasströmung benötigt wird, um das vernebelte Medium zu fördern, beispielsweise durch ein Rohrleitungssystem, ist es besonders zweckmäßig, wenn der Sprühstrahl durch eine drucklos-mechanische Zerstäubung, beispielsweise über einen sogenannten Schwingzerstäuber erfolgt.

Die Erfindung wird anhand schematischer Zeichnungen für ein Ausführungsbeispiel näher erläutert. Es zeigen:
- Fig. 1: eine Whirlpoolwanne mit Dampfdesinfektion,
- Fig. 2: eine Einrichtung zur Erzeugung eines aufgeheizten nebelförmigen Mediums.

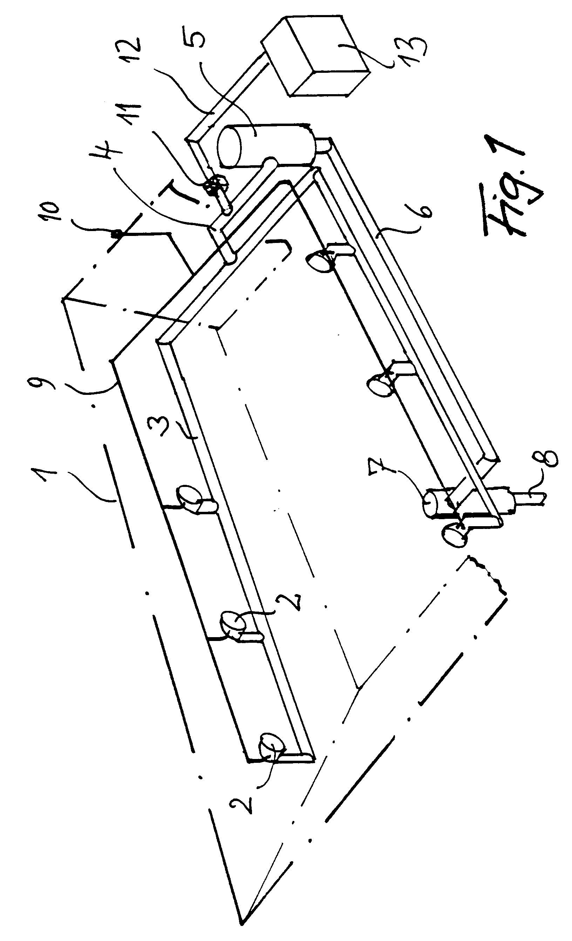

Die in Fig. 1 nur angedeutete Whirlpool-Wanne 1 ist in ihren Seitenwandungen mit mehreren Wirbeldüsen 2 versehen, die über eine Druckleitung 3 mit der Druckleitung 4 einer Umwälzpumpe 5 in Verbindung stehen. Die Umwälzpumpe 5 ist über eine Saugleitung 6 an eine im Wannenboden befindliche kombinierte Ansaug-Ablauföffnung 7 angeschlossen. Die kombinierte Ansaug-Ablauföffnung ist so ausgebildet, daß im Betrieb über die Saugleitung 6 aus dem Wanneninnenraum Wasser angesaugt und über die Wirbeldüsen 2 wieder in den gefüllten Wanneninnenraum unterhalb der Wasseroberfläche eingeleitet wird. Wird die Ablauföffnung geöffnet, dann kann das Wasser sowohl aus dem Wanneninnenraum als auch aus dem durch die Rohrleitung 3, die Druckleitung 4 und die Seigleitung 6 gebildeten Rohrsystem vollständig in das Ablaßrohr 8 ablaufen.

Die Wirbeldüsen 2 sind ferner an eine Luftleitung 9 abgeschlossen, die eine im Bereich des Wannenrandes angeordnete einstellbare Ansaugöffnung 10 aufweist, so daß beim Betrieb der Umwälzpumpe 5 über den aus den Wirbeldüsen 2 austretenden Wasserstrahl Luft angesaugt und in den Wanneninnenraum ein Wasser-Luft-Gemisch strahlförmig eingedrückt wird.

Nach Beendigung des Bades wird die Ablauföffnung 8 geöffnet, so daß das Wasser vollständig auslaufen kann.

An die Druckleitung 4 ist nun über ein zwischengeschaltetes Sperrventil 11 eine Rohrleitung 12 angeschlossen, die mit einer Einrichtung 13 zur Erzeugung eines aufgeheizten nebelförmigen Mediums, beispielsweise einem elektrisch beheizbaren Dampfgenerator 13 verbunden ist. Sobald nun die Wanne einschließlich ihres Rohrsystems entleert ist, wird das Sperrventil geöffnet und der Dampfgenerator in Betrieb gesetzt. Der im Dampfgenerator erzeugte Dampf drückt nun durch die Rohrleitung 12 in die Druckleitung 4 des Rohrsystems und strömt dann über die Rohrleitung 3 und die Wirbeldüsen 2 in den Wanneninnenraum. Der Strömungswiderstand bewirkt jedoch, daß auch ein Teil des Dampfes über die als Kreiselpumpe ausgebildete Umwälzpumpe und die Saugleitung 6 in die nunmehr geöffnete Ansaug-Ablauf-Armatur 7 einströmt und hierbei aus der offenen Ablauföffnung im Wannenboden ebenfalls ausströmen kann.

Hierbei werden nun alle Bereiche des Rohrsystems einschließlich der Düsenmündungen und der Umwälzpumpe vom Dampf berührt, so daß aus dem verbrauchten Badewasser im Rohrleitungssystem zurückgebliebene Keime schon nach kurzer Zeit abgetötet werden. Bei entsprechender Ausrüstung der Wanne mit Werkstoffen, die bis in den Bereich von etwa über 100°C temperaturbeständig sind, kann hier mit einer Ausgangsdampftemperatur von 100°C gearbeitet werden, wobei schon nach kurzer Zeit auch die vom Einleitungspunkt am weitesten entfernt liegenden Wirbeldüsen einer Temperatur von über 90°C ausgesetzt sind und somit eine Abtötung etwa vorhandener Keime mit Sicherheit gewährleistet ist.

Hierbei ist zu berücksichtigen, daß in der Regel für das Rohrsystem die zugehörigen Fittings und Düsen aus Kunststoffen bestehen, die einer Temperaturbelastung bis etwas über 100°C standhalten.

Der Dampfgenerator 13 benötigt für diesen Einsatzzweck eine elektrische Anschlußleistung von max. 3 kW, wobei für eine einwandfreie Desinfektion eine Wassermenge von max. 3 l zu verdampfen ist.

Bei Ausführungsformen, die zusätzlich oder anstelle der Wirbeldüsen 2 noch mit Bodendüsen versehen sind, über die ausschließlich Luft eingeleitet wird, ist ein entsprechender Anschluß des Dampfgenerators 13 an diese luftführende Leitung unmittelbar hinter dem Gebläse vorzusehen, so daß auch diese Leitungen, die bei abgeschaltetem Gebläse oder auch beim Entleeren mit dem Wasser in Berührung kommen, ebenfalls mit dem heißen Dampf in Berührung kommen.

Für beide Ausführungsformen kann es zweckmäßig sein, wenn ein zusätzlicher Frischwasseranschluß vorgesehen ist, so daß mit frischem Leitungswasser unmittelbar nach dem Ablassen des verbrauchten Badewassers das Rohrleitungssystem durchspült werden kann, um hier zunächst einmal mechanisch etwaige Ablagerungen wie Hautschuppen, Seifen und Schmutzreste oder dergl. auszuschwemmen. Die so mit Klarwasser praktisch drucklos durchspülten Leitungen werden anschließend, wie vorstehend beschrieben, mit Dampf als nebelförmigem Medium beaufschlagt.

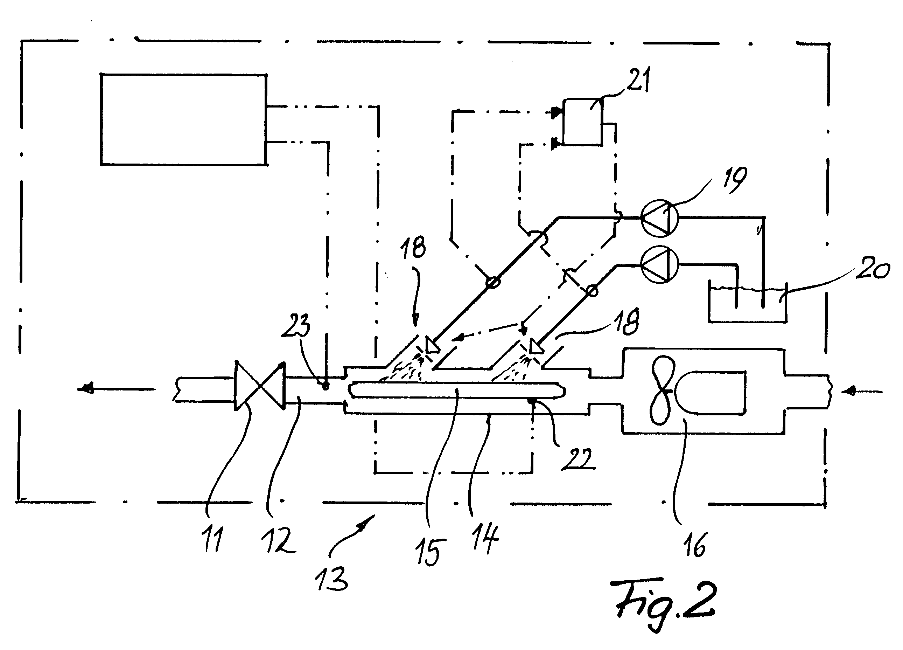

In Fig. 2 ist eine spezielle Ausführungsform der Einrichtung 13 dargestellt. Diese weist eine Verdampferkammer 14 auf, in der eine beheizbare Verdampferfläche 15 angeordnet ist. In diese Verdampferkammer 14 mündet der Auslaß eines Gebläses 16 ein, so daß mit Luft als Trägergas die Verdampferkammer 14 durchströmt werden kann. Der über das Sperrventil 11 absperrbare Auslaß 17 der Verdampferkammer 14 ist an das zu bedampfende System, beispielsweise die in Fig. 1 dargestellte Whirlpoolwanne angeschlossen.

In die Verdampferkammer 14 mündet wenigstens eine Sprüheinrichtung 18 ein, deren Sprühstrahl gegen die Verdampferfläche 15 gerichtet ist. Zur Vereinfachung einer Mengenregelung sind hier zwei Sprüheinrichtungen 18 angeordnet. Diese können entweder als Druckluftsprüheinrichtung, bevorzugt aber als drucklos-mechanische Sprüheinrichtung, beispielsweise als Schwingzerstäuber ausgebildet sein. Bei dieser Ausführung wird die zu zerstäubende Flüssigkeit aus einem höherliegenden Behälter oder, wie hier dargestellt, mittels Förderpumpe 19 aus einem Behälter 20 dem System zugeführt. Über einen Wächter 21, der mit der Energieversorgung des Schwingzerstäubers 18 in Verbindung steht, ist sichergestellt, daß dieser nur bei gefüllter Zuleitung eingeschaltet werden kann bzw. bei Wassermangel abschalten kann. Die Mengenregulierung kann bei einem Schwingzerstäuber unmittelbar über eine Verstellung der Amplitude des Schwingorgans erreicht werden.

Die Temperatur der Verdampferfläche 15 einerseits und des erzeugten nebelförmigen Mediums andererseits wird über Temperaturfühler 22 und 23 erfaßt und dient der Regelung der zuzuführenden Flüssigkeitsmenge und/oder der Temperatur der Verdampferfläche, so daß die gewünschte Temperatur eingehalten und/oder eine Überhitzung ausgeschlossen werden kann. Falls eine höhere Temperatur im Bedampfungsbereich gewünscht wird, kann dies über eine entsprechende Aufheizung des Trägergases erreicht werden, wobei dann allerdings die eingesprühte Flüssigkeit nicht mehr als sichtbarer Nebel bzw. Dampf im physikalischen Sinn abgegeben wird.

Die anhand von Fig. 2 beschriebene Einrichtung kann auch zur Bedampfung anderer sanitärer Anlagen verwendet werden. Bei entsprechender Auslegung der Heiz- und Verdampferleistung sowie der Zerstäuberleistung ist auch der Einsatz zur Bedampfung einer Dampfbadekabine möglich, da auch hier eine Grenze in der maximal zulässigen Temeratur einzuhalten ist.

## Patentansprüche

1. Verfahren zur Desinfektion des Rohrsystems an einem Whirlpool durch ein aufgeheiztes strömungsfähiges Medium, das durch das Rohrsystem hindurchgeleitet wird, **dadurch gekennzeichnet**, daß ein nebelförmiges Medium durch Verdampfen einer Flüssigkeit unter Temperatureinwirkung und unter normalem atmosphärischem Druck durch Einbringen in feinster Verteilung zur Bildung eines Aerosols in ein Trägergas erzeugt wird.

2. Verfahren nach Anspruch 1, dadurch gekennzeichnet, daß zur Verdampfung die Flüssigkeit auf eine vorzugsweise temperaturregelbare Verdampferfläche aufgesprüht wird.

3. Verfahren nach Anspruch 1 oder 2, dadurch gekennzeichnet, daß das Trägergas vor, spätestens bei Bildung des Aerosols aufgeheizt wird.

4. Verfahren nach Anspruch 1 bis 3, dadurch gekennzeichnet, daß das gebildete Aerosol vor dem Kontakt mir der zu desinfizierenden Oberfläche aufgeheizt wird.

5. Verfahren nach einem der Ansprüche 1 bis 4, dadurch gekennzeichnet, daß als Flüssigkeit zur Erzeugung des nebelförmigen Mediums keimfreies Wasser verwendet wird.

6. Verfahren nach Anspruch 1 bis 5, dadurch gekennzeichnet, daß als Flüssigkeit zur Erzeugung des nebelförmigen Mediums, zumindest teilweise ein temperaturbeständiges Desinfektionsmittel verwendet wird.

7. Verfahren nach einem der Ansprüche 1 bis 6, dadurch gekennzeichnet, daß die einzusprühende Flüssigkeitsmenge bei vorgegebener Temperatur des Trägergases und/oder der Heizleistung der Heizoberfläche in Abhängigkeit von der Temperatur des nebelförmigen Mediums hinter der Einsprühstelle geregelt wird.

8. Whirlpool-Anlage zur Durchführung des Verfahrens nach den Ansprüchen 1 bis 7, mit einem Becken, dessen Wandungen und/oder Boden mit Düsen zur Einleitung von Wasser und/oder Luft versehen sind, die über Rohrleitungen mit wenigstens einem Druckerzeuger zur Beaufschlagung der Düsen mit Wasser und/oder Luft verbunden sind, dadurch gekennzeichnet, daß an eine zentrale Druck- und/oder Saugleitung (3, 6) im Bereich des Druckerzeugers (5) eine Zuleitung (12) einmündet, die mit einer Einrichtung (13) zur Erzeugung eines aufgeheizten nebelförmigen strömungsfähigen Mediums verbunden ist, die durch ein an die Zuleitung (12) angeschlossenes Gehäuse (14), das mit wenigstens einem Gebläse (16) und mit wenigstens einer Heizeinrichtung (15) zur Aufheizung von Gebläseluft in Verbindung steht, und wenigstens eine Sprühvorrichtung (18) zur Erzeugung feiner Flüssigkeitströpfchen, die in das Gehäuse (14) im Bereich der Heizeinrichtung (15) mündet.

9. Whirlpool-Anlage nach Anspruch 8, dadurch gekennzeichnet, daß der Sprühstrahl gegen eine Verdampferfläche der Heizeinrichtung (15) gerichtet ist.

10. Whirlpool-Anlage nach Anspruch 8 oder 9, dadurch gekennzeichnet, daß die Sprühvorrichtung (18) als drucklos-mechanische Zerstäubervorrichtung, insbesondere als Schwingzerstäuber, ausgebildet ist.

11. Whirlpool-Anlage nach einem der Ansprüche 8 bis 10, dadurch gekennzeichnet, daß zur Regelung der einzusprühenden Flüssigkeitsmenge und/oder der Temperatur die Heizeinrichtung (15) mit einem Temperaturfühler (22) in Verbindung steht und daß ein Temperaturfühler (23) im Bereich der Zuleitung (12) angeordnet ist und daß beide Temperaturfühler mit einer Regeleinrichtung zur Veränderung der Sprühflüssigkeitsmenge verbunden sind.

## Claims

1. A method for disinfecting the pipe system of a whirlpool bath by a heated, flowable medium which is passed through the pipe system, characterised in that a mist-like medium is produced by evaporating a liquid under the action of temperature and under normal atmospheric pressure by introduction into a carrier gas in a very fine distribution to form an aerosol.

2. A method according to Claim 1, characterised in that for evaporation the liquid is sprayed on to an evaporator surface which is preferably of controllable temperature.

3. A method according to Claim 1 or 2, characterised in that the carrier gas is heated prior to, or at the latest upon, formation of the aerosol.

4. A method according to Claims 1 to 3, characterised in that the aerosol formed is heated before contact with the surface to be disinfected.

5. A method according to one of Claims 1 to 4, characterised in that sterilised water is used as liquid for producing the mist-like medium.

6. A method according to Claims 1 to 5, characterised in that a heat-resistant disinfectant is used at least in part as the liquid for producing the mist-like medium.

7. A method according to one of Claims 1 to 6, characterised in that the quantity of liquid to be sprayed in at a predetermined temperature of the carrier gas and/or the heating capacity of the heating surface is regulated dependent on the temperature of the mist-like medium behind the point at which it is sprayed in.

8. A whirlpool system for performing the method according to Claims 1 to 7, comprising a basin, the walls and/or base of which are provided with nozzles for introducing water and/or air, which are connected via pipes to at least one pressure generator for supplying the nozzles with water and/or air, characterised in that a feed line (12) opens into a central pressure and/or suction line (3, 6) in the region of the pressure generator (5), which line is connected to a means (13) for producing a heated, mist-like flowable medium which is connected by a housing (14) connected to the feed line (12), which housing is connected to at least one fan (16) and to at least one heating means (15) for heating fan air, and at least one spraying device (18) for producing fine liquid droplets, which opens into the housing (14) in the region of the heating means (15).

9. A whirlpool system according to Claim 8, characterised in that the spray jet is directed against an evaporator surface of the heating means (15).

10. A whirlpool system according to Claim 8 or 9, characterised in that the spraying device (18) is designed as a non-pressurised mechanical atomiser device, in particular as an oscillating atomiser.

11. A whirlpool system according to one of Claims 8 to 10, characterised in that for controlling the quantity of liquid to be sprayed in and/or the temperature the heating means (15) is connected to a temperature sensor (22) and that a temperature sensor (23) is arranged in the region of the feed line (12) and that the two temperature sensors are connected to a regulating means for changing the quantity of spraying liquid.

## Revendications

1. Procédé pour la désinfection du système de tuyauterie d'un bain à remous au moyen d'un fluide chauffé qui passe à travers le système de tuyauterie, caractérisé en ce que l'on produit un fluide sous forme de brouillard en vaporisant un liquide sous l'action de la température et sous la pression atmosphérique normale en le transformant en une très fine dispersion pour former un aérosol dans un gaz porteur.

2. Procédé selon la revendication 1, caractérisé en ce que, pour la vaporisation, on pulvérise le liquide sur une surface de vaporisateur dont la température est de préférence réglable.

3. Procédé selon l'une des revendications 1 ou 2, caractérisé en ce que l'on chauffe le gaz porteur avant ou au plus tard lors de la formation de l'aérosol.

4. Procédé selon l'une des revendications 1 à 3, caractérisé en ce que l'on chauffe l'aérosol formé avant le contact avec la surface à désinfecter.

5. Procédé selon l'une des revendications 1 à 4, caractérisé en ce que l'on utilise comme liquide pour la production du milieu sous forme de brouillard de l'eau stérile.

6. Procédé selon l'une des revendications 1 à 5, caractérisé en ce que l'on utilise comme liquide pour la production du milieu sous forme de brouillard au moins en partie un produit désinfectant résistant à la chaleur.

7. Procédé selon l'une des revendications 1 à 6, caractérisé en ce que l'on règle la quantité de liquide à pulvériser, selon la température déterminée du gaz porteur et/ou selon la puissance de chauffage de la surface de chauffage, en fonction de la température du milieu sous forme de brouillard après le lieu de pulvérisation.

8. Installation de bain à remous pour la mise en oeuvre du procédé selon l'une des revendications 1 à 7, comportant un bassin dont les parois et/ou le fond sont munis de buses qui sont destinées à l'arrivée d'eau et/ou d'air et qui sont reliées, par l'intermédiaire de tuyaux, à au moins un générateur de pression destiné à alimenter les buses en eau et/ou en air, caractérisée en ce qu'une conduite d'alimentation (12) débouche dans une conduite centrale de pression et/ou d'aspiration (3, 6) dans la zone du générateur de pression (5), ladite conduite d'alimentation (12) étant reliée à un dispositif (13) qui est destiné à la production d'un milieu sous forme de brouillard chauffé et qui est constitué d'une chambre (14), raccordée à la conduite d'alimentation (12) et reliée à au moins un ventilateur (16) et à au moins un dispositif de chauffage (15) pour chauffer l'air soufflé, et d'au moins un dispositif de pulvérisation (18) destiné à produire de fines gouttelettes de liquide et débouchant dans la chambre (14) dans la zone du dispositif de chauffage (15).

9. Installation de bain à remous selon la revendication 8, caractérisée en ce que le jet de pulvérisation est dirigé sur une surface de vaporisateur du dispositif de chauffage (15).

10. Installation de bain à remous selon l'une des revendications 8 ou 9, caractérisée en ce que le dispositif de pulvérisation (18) est constitué par un dispositif pulvérisateur mécanique sans pression, notamment par un pulvérisateur à vibrations.

11. Installation de bain à remous selon l'une des revendications 8 à 10, caractérisée en ce que, pour le réglage de la quantité de liquide à pulvériser et/ou de la température, le dispositif de chauffage (15) est relié à un capteur de température (22), qu'un capteur de température (23) est agencé dans la zone de la conduite d'alimentation (12) et que les deux capteurs de température sont reliés à un dispositif de réglage destiné à la modification de la quantité de liquide à pulvériser.
